# EUROPEAN PATENT APPLICATION

(11) **EP 2 338 876 A1**
(43) Date of publication of application: **29.06.2011**
(21) Application number: 09180329.6
(22) Date of filing: 22.12.2009
(51) Int. Cl.: C07C 303/40, C07C 303/44, C07C 311/37

(54) **Tamsulosin adipate for pharmaceutical use**

(71) Applicant: LEK Pharmaceuticals d.d., 1526 Ljubljana (SI); Hexal AG, 83607 Holzkirchen (DE)
(72) Inventor: Furlan, Borut, 1526 Ljubljana (SI); Naversnik, Klemen, 1526 Ljubljana (SI); Ham, Zoran, 1526 Ljubljana (SI); Obermeier, Petra, 83607 Holzkirchen (DE); Oelke, Rainer, 83607 Holzkirchen (DE); Wagner, Per, 83607 Holzkirchen (DE)

(57) **Abstract**

The present invention belongs to the field of organic chemistry and relates to tamsulosin adipate and the manufacturing process thereto.

## Description

### Field of the invention

The present invention belongs to the field of organic chemistry and relates to a new form of tamsulosin for pharmaceutical use and the manufacturing process thereto. More specifically the present invention relates to the reaction of tamsulosin base with adipic acid to yield tamsulosin adipate.

### Background of the invention

(R)-5-(2-(2-(2-ethoxyphenoxy)ethylamino)propyl)-2-methoxybenzenesulphonamide (formula 1),designated with the generic pharmaceutical name tamsulosin, has been used in the form of the hydrochloride salt of the pure R-enantiomer for the treatment of benign prostatic hyperplasia.

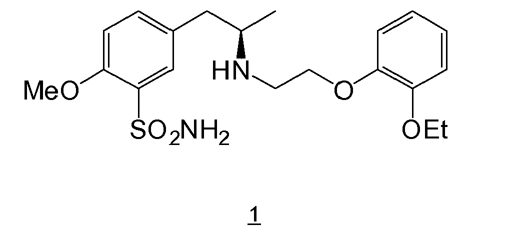

For incorporation into a pharmaceutical formulation, pharmaceutical active substances must show defined desired physico-chemical properties, such as solubility in water and certain other media, suitable particle size, stability and non-hygroscopicity. All of these properties could be taken into consideration when selecting pharmacologically the most appropriate form. Thereby the optimal bioavailability can be achieved.

Tamsulosin is administered orally in the form of tablets or capsules. It is wishful for the indication of BPH that the level of the ingredient is as constant as possible for all day and the administration is as rare as possible, for instance once per day. Extended release oral formulations have therefore been developed. The hydrochloride salt exhibits very useful properties for oral use; in the enteric pH, the salt is neutralised and tamsulosin free base is absorbed with a good bioavailability. Various approaches for very prolonged action often use lipid matrix for manufacturing of final dosage forms. For instance depo intramuscular injection may contain lipid ingredients or liposomes, some other final forms, such as suppositoria also contain predominantly lipid material. Some modern approaches, such as inhalable, sublingual, buccal or intradermal uses demand passing lipid physiological barriers but there is not enough opportunity for neutralisation of a hydrophyllic salt which is not able to cross the barrier. As a salt of a strong acid the hydrochloride salt of tamsulosin is not very appropriate for such application because the molecule of tamsulosin is protonated and as a cation it is too polar for smooth passing lipid barriers. Tamsulosin base may be an option for a final dosage forms for such purpose but due to a low water solubility thereof it may cause some problems if there is also a need for contacts with hydrophilic surface during therapeutic application.

Therefore there is a great need to find a form of tamsulosin which would smoothly interact with hydrophilic and lipophilic surfaces.

It was surprisingly found that many tamsulosin salts are mentioned in literature as pharmaceutically applicable ones (US 2005/106253, WO 05/053659) but only few are prepared and characterized ― oxalate, maleate, fumarate, tartrate (US 7238839), while mandelate (WO 06/004093) and camsylate salts (WO 03/037850, WO 03/037851) are used only for enantiomeric separation. None of them is mentioned to have beneficial effects for pharmaceutical use where interactions with lipids take place.

### Summary of the inventon

The first embodiment of the present invention provides the substance of tamsulosin adipate characterized by structure and ¹H-NMR.

Another embodiment of this invention provides a reaction of process for the preparation of tamsulosin adipate by dissolving tamsulosin base and adipic acid in a solvent of a medium polarity.

Another embodiment of this invention provides anhydrous crystalline tamsulosin adipate characterized by X-ray powder diffractometry, DSC and IR.

Another embodiment of this invention provides a reaction of process for the preparation of tamsulosin adipate by dissolving tamsulosin base and adipic acid in an anhydrous solvent of a medium polarity, such as ketones, nitriles and esters, e.g. acetone, acetonitrile, ethyl acetate.

Another embodiment of this invention provides crystalline tamsulosin adipate dihydrate characterized by X-ray powder diffractometry, DSC and IR.

Another embodiment of this invention provides a reaction of process for the preparation of tamsulosin adipate dihydrate by dissolving tamsulosin base and adipic acid in a wet solvent of a medium polarity.

Another embodiment of this invention provides a reaction of process for the preparation of tamsulosin adipate dihydrate by conversion of anhydrous tamsulosin adipate by exposing to air humidity at least 50 %.

Another embodiment of this invention provides amorphous tamsulosin adipate dihydrate characterized by plain curve in X-ray powder diffractometry.

Another embodiment of this invention provides a reaction of process for the preparation of amorphous tamsulosin adipate by exposing tamsulosin adipate dihydrate to air humidity below 20 %.

Another embodiment of this invention provides a pharmaceutical formulation comprising a pharmaceutically effective amount of tamsulosin adipate.

Another embodiment of this invention provides a use of tamsulosin adipate for prevention and/or treatment of benign prostatic hyperplasia, urinary incontinence and related conditions and disorders.

Another embodiment of this invention provides a use of tamsulosin adipate for the preparation of pharmaceutical formulations.

### Brief description of the drawings

Figure 1 is a ¹H-NMR spectrum of crystalline tamsulosin adipate.
Figure 2 is an X-ray powder diffraction pattern of anhydrous crystalline tamsulosin adipate.
Figure 3 shows a DSC thermogram of anhydrous crystalline tamsulosin adipate.
Figure 4 shows an IR spectrum of crystalline tamsulosin adipate.
Figure 5 is an X-ray powder diffraction pattern of crystalline tamsulosin adipate dihydrate.
Figure 6 shows a DSC thermogram of crystalline tamsulosin adipate dihydrate.
Figure 7 shows an IR spectrum of crystalline tamsulosin adipate dihydrate.
Figure 8 is an X-ray diffraction pattern of crystalline tamsulosin adipate dihydrate prepared as a monocrystal
Figure 9 is an X-ray powder diffraction pattern of amorphous tamsulosin adipate dihydrate.
Figure 10 shows a DSC thermogram of amorphous tamsulosin adipate.
Figure 11 shows an IR spectrum of amorphous tamsulosin adipate.

### Detailed description of the invention

Middle and higher aliphatic acids are comprehensive compounds in the pharmaceutical industry used mainly as excipients for various purposes such as lubricants, emulsifiers, plasticizers, flavouring agents. These pharmaceutically acceptable middle and higher aliphatic acids include C₅-C₂₄ linear saturated aliphatic acids, preferably lauric, palmitic and stearic acid, C₅-C₂₄ branched saturated aliphatic acids, such as 2-ethylhexanoic acid, cyclic aliphatic acids such as cyclohexane carboxylic acid, C₅-C₂₄ alkenoic acids, preferably oleic acid, partially substituted C₅-C₂₄ aliphatic acid, preferably hydroxy substituted, such as ricinoleic acid, C₅-C₂₄ aliphatic dicarboxylic acids, preferably pentanedioic (glutaric), hexanedioic (adipic), octanedioic (suberic), decanedioic (sebacic) acid. They are rarely used as counter-anions in active pharmaceutical ingredients. Indeed while combining an active basic molecule and a higher aliphatic acid due to its emulsifying properties and weak acidic character of carboxylic groups usually foams or oils are formed. For instance, when evaporating methanol from a solution of tamsulosin base and adipic acid, a foam residue is formed which slowly solidifies into a plastic-like hard coat (see Reference example 1). Such material is difficult to isolate and difficult to handle in the industrial level. It is useful only in the case when its formation is a part of final dosage form preparation in which other components are added simultaneously. But if the mother molecule is highly crystalline such as in the case of tamsulosin the base can be precipitated from a homogenous mixture of an acid and the base in a form of particles (see Reference example 2). Such mixture of the base and its salt is not repeatable enough to satisfy regulatory demands for final dosage preparation. In such cases there is strong need for handable solid, powder like salt. We surprisingly found that after a special treatment of tamsulosin and middle and higher aliphatic acids in selected solvents, an easy isolable powder like, preferably crystaline material is formed.

In one embodiment of the invention tamsulosin base is dissolved in a solvent of medium polarity or in a mixture thereof and while stirring a middle or a higher aliphatic acid is added in pure state (solid or neat liquid) or dissolved in a same or different solvent of medium polarity or optionally in a minimum amount of a solvent other than the solvent of medium polarity. The acid is added in one portion, portionwise or continuously. The mixture is then stirred or let to stand for 1 hour to 10 days and the solid precipitate is filtered off and dried to remove residual solvents. The precipitation is optionally induced by adding seeds in order to guarantee more controlled and repeatable precipitation.

A solvent of medium polarity means in this case a solvent selected from C₃-C₈ ketones, preferably acetone, C₂-C₄ nitriles, preferably acetonitrile, C₃-C₈ esters, preferably ethyl acetate and isopropyl acetate, and C₅-C₆ cyclic ethers, preferably tetrahydrofurane, and excludes protic solvents such alcohols and highly solubilising solvents such as chlorinated hydrocarbons and amides in a polar side and acyclic ethers and hydrocarbons in an apolar side. Apolar solvents can be optionally used as cosolvents after the addition of the acid in order to enhance the yield but in a limited extent to avoid liquefying the precipitate, the preferred antisolvents are acyclic ethers, preferably diethyl ether or *tert*-butyl methyl ether.

A solvent can be optionally water wet if the salt forms crystalline hydrate. The water contain can be from 0,1 % (w/w) to saturation.

The middle or higher aliphatic acid is selected from C₅-C₂₄ linear saturated aliphatic acids, preferably lauric, palmitic and stearic acid, C₅-C₂₄ branched saturated aliphatic acids, such as 2-ethylhexanoic acid, cyclic aliphatic acids such as cyclohexane carboxylic acid, C₅-C₂₄ alkenoic acids, preferably oleic acid, partially substituted C₅-C₂₄ aliphatic acid, preferably hydroxy substituted, such as ricinoleic acid, C₅-C₂₄ aliphatic dicarboxylic acids, preferably pentanedioic (glutaric), hexanedioic (adipic), octanedioic (suberic), decanedioic (sebacic) acid.

Tamsulosin base is prepared according to any synthetic procedure described in literature and optionally purified by recrystallization, alternatively it can be prepared from commercial tamsulosin hydrochloride by neutralization, preferably by ammonia solution with further isolation and optional purification.

The seeds for inducing precipitation may involve particles of the expecting salt or other inert particles. The first crop of seeds of the expecting salt may be prepared by routine precipitation in a smaller laboratory scale, optionally if precipitation is not spontaneous the seeds could be prepared by a help of well known laboratory tricks such as scratching the wall of the reaction vessel, slow spontaneous evaporation of a solvent, standing the solution for a longer period, adding various antisolvents, ultrasound induction or they have appeared in an accidental experiment.

The isolated solid powder like tamsulosin salt can be optionally recrystallised from a medium including an organic solvent and/or water.

In a special embodiment the present invention relates to a disclosure of a crystalline tamsulosin adipate (tamsulosin hexanedioate) and the preparation thereof. Crystalline tamsulosin adipate according to our invention is a solid adduct between tamsulosin and adipic acid in which physical properties of particular components are not detectable and in which bimolecular interactions may be polar (cation-anion interactions as in typical salts) or may be induced by hydrogen bonds (as in co-crystals).

Tamsulosin adipate may be prepared by mixing tamsulosin base and adipic (hexanedioic) acid. The formation of crystals is carried out in a solvent of a medium polarity selected from C₃-C₈ ketones, preferably acetone, C₂-C₄ nitriles, preferably acetonitrile, C₃-C₈ esters, preferably ethyl acetate and isopropyl acetate, the most preferred solvent is ethyl acetate. Preferably tamsulosin base is dissolved in a solvent of a medium polarity, heated to the boiling point and adipic acid is added either as a solid or dissolved or suspended in the same or different solvent of a medium polarity, most preferably in the solid state. The precipitation of adipate occurs after cooling the stirred mixture from the heated solution, usually below 50°C, preferably to room temperature or lower. In another embodiment, the crystalline adipate is formed by precipitation when adding an antisolvent preferably selected from ethers, most preferably from diethyl ether or methyl-tert-butyl ether, or the antisolvent is added after the precipitation from the main solvent in order to improve the yield of the precipitate. Optionally the seeds of tamsulosin adipate may be added during cooling of the solution.

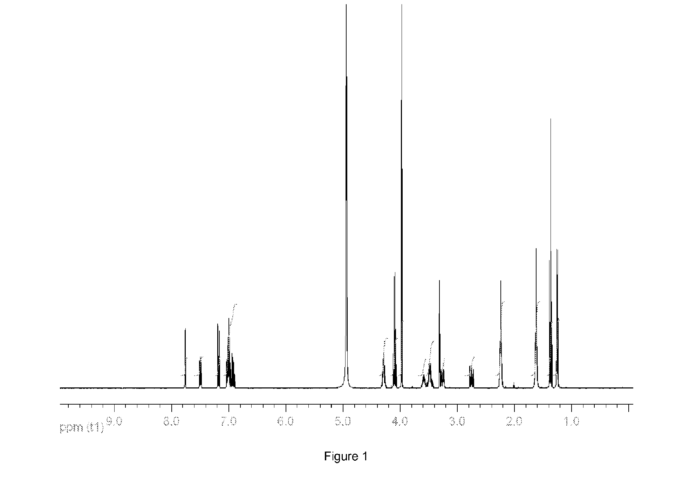

Tamsulosin adipate prepared by the procedure of the invention consists of the tamsulosin part and the adipic part in the ratio of 1:1 (Formula 2) as it is shown in NMR spectrum of Figure 1.

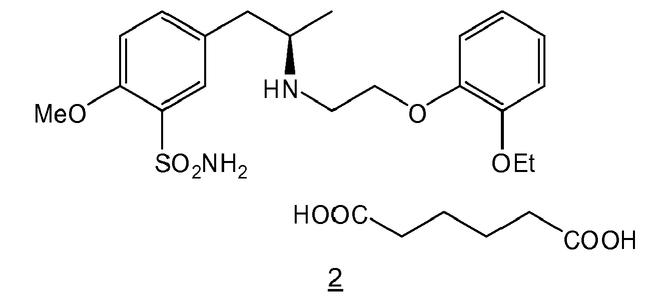

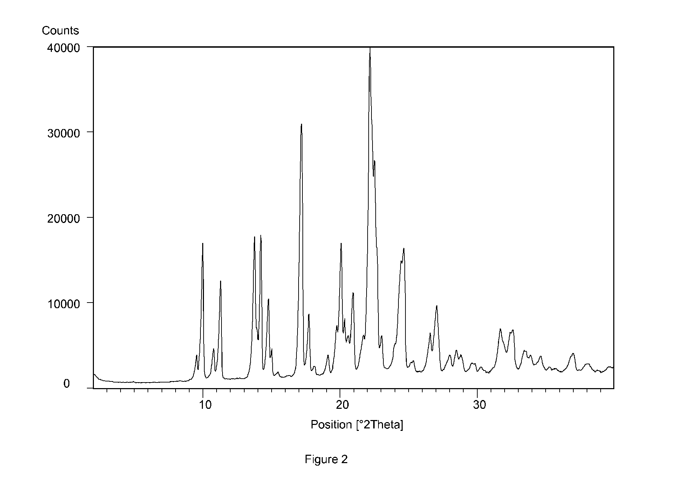

Tamsulosin adipate prepared by the process of the invention using anhydrous said solvents exhibits clearly a crystalline state shown by sharp peaks in XRPD (Figure 2). It is characterized by the peaks at about 2θ = 10.0, 11.3, 13.8, 14.2, 17.2, 22.2, and 24.7°, particularly characteristic peaks are 11.3, 17.2, 22.2 °. Such crystalline state is characteristic for anhydrous crystalline tamsulosin adipate.

The term "anhydrous solvent" means in this case a commercially available solvent without any additional amount of water.

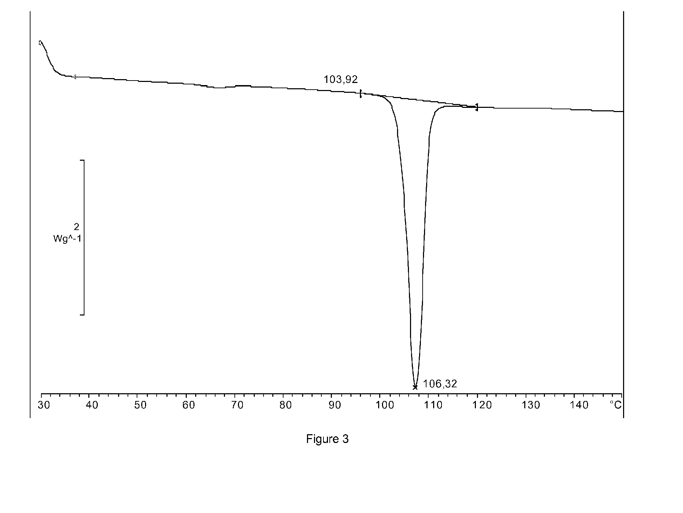

Anhydrous crystalline tamsulosin adipate samples prepared by the process of the invention show melting points within the range of 93-97 °C, if visually detected by using the Kofler microstage. If differential scanning calorimetry (DSC) is used, melting points within the range of 101-106 °C, more narrowly at 103-104 °C, are detected as an onset of the endothermic transformation ― Figure 3.

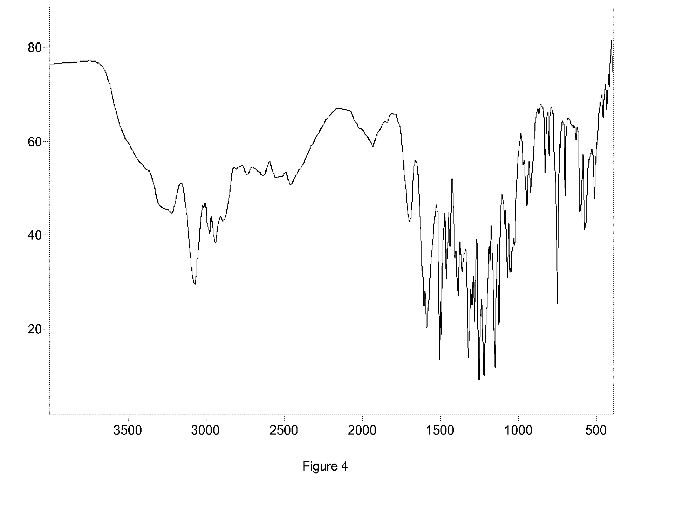

Tamsulosin adipate prepared by the process of the invention shows characteristic IR spectrum as shown in Figure 4 having characteristic bands at 3069, 1590, 1507 1253, 1221, 1150, 754 cm -1.

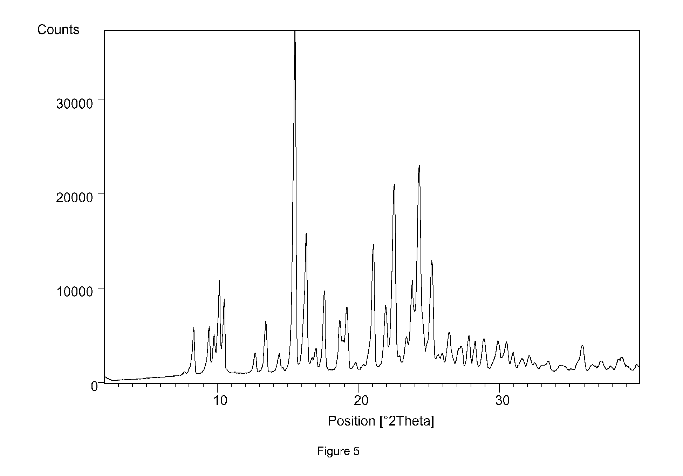

Tamsulosin adipate prepared by the process of the invention using wet said solvents exhibits clearly a crystalline state shown by sharp peaks in XRPD (Figure 5). It is characterized by the peaks at about 2θ = 10.2, 10.5, 13.5, 15.5, 16.3, 21.1, 21.9° 24.3, 25.2, 28.3 and 35.9°, particularly characteristic peaks are 15.5, 21.9°, 28.3, and 35.9°. Such crystalline state is characteristic for tamsulosin adipate dihydrate.

The term wet solvent means in this case a commercially available solvent with additional amount of water added. The additional amount of water is added to reach a concentration from 0.5 % (v/v) to level of saturation in the solvent of preparation or a concentration from 0.25 % (v/v) to level of saturation in the final solvent/antisolvent mixture.

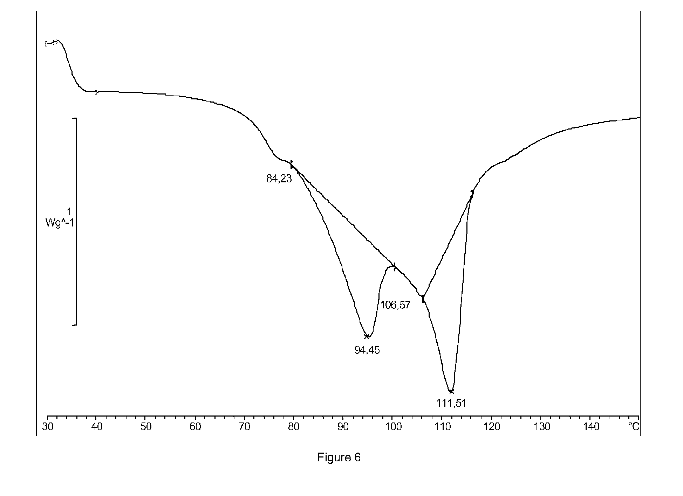

Tamsulosin adipate dihydrate samples prepared by the process of the invention show melting points within the range of 78-92 °C, preferably in the range of 88-90 °C, if visually detected by using the Kofler microstage, but this value may represent water removal or conversion of dihydrate to anhydrous form. If differential scanning calorimetry (DSC) is used two endothermic transformation are found, an endothermic peak is identified as an onset in the range between 70 and 90 ° which may represent water removing or dihydrate transformation, another one is detected as an onset within the range of 100-110 °C, more narrowly at 103-105 °C which may represent a melting point of anhydrous material or glass transition transformation of amorphous form or a mixture of both effects - Figure 6. The intensity of the peak may vary from the level of just detected to the level higher than peak level of the melting point what depends on underhydration-overhydration level or degree of crystallinity.

Tamsulosin adipate dihydrate contains theoretically 5.9 % (w/w) of water, but experimentally from 5-7 %, preferably 5.5-6.5 % most preferably 5.7- 6.1 % of water.

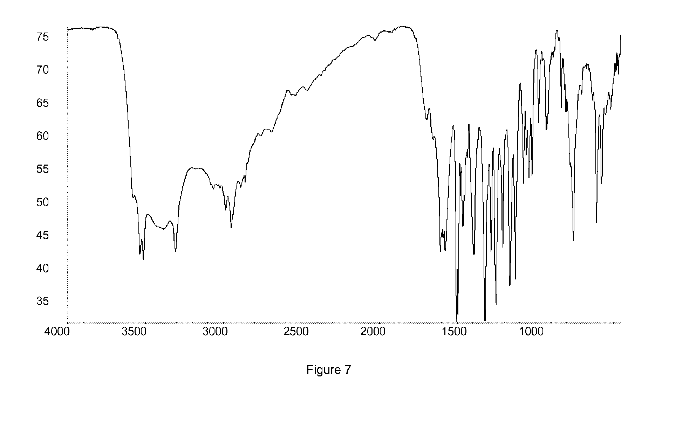

Tamsulosin adipate dihydrate prepared by the process of the invention shows characteristic IR spectrum as depicted in Figure 7.

In another embodiment of the invention tamsulosin adipate dihydrate is prepared by exposing anhydrous crystalline tamsulosin adipate to air moisture above 50 % of relative moisture, preferably above 60 %, most preferably above 70 %. Incorporation of crystal water is carried out at temperatures bellow 60 °C and is finished in few hours to 4 weeks depending on degree of relative moisture. Thus in a standard stress stability test in open dish at 40 °C and 75 % of relative moisture for 14 days anhydrous crystalline tamsulosin adipate is completely transformed to tamsulosin adipate dihydrate with identical XRPD data as the dihydrate prepared from wet solvents.

In another embodiment of the invention tamsulosin adipate dihydrate can be prepared also from water solutions from more soluble forms of tamsulosin adipate (anhydrous or amorphous). Depending on kinetic factors two polymorphs of tamsulosin adipate dihydrate can be obtained.

In another embodiment tamsulosin adipate (anhydrous or amorphous) is dissolved in a small amount of water, preferably in 60-100 ml of water per gram of the salt, most preferably in 80-90 ml per gram, preferably at room temperature. Less soluble dihydrate spontaneously precipitates from the solution, yielding tamsulosin adipate dihydrate, characterized by the peaks at about 2θ= 10.2, 10.5, 13.5, 15.5, 16.3, 21.1, 21.9° 24.3, 25.2, 28.3 and 35.9° particularly characteristic peaks are 15.5, 21.9°, 28.3, and 35.9°. The polymorph named tamsulosin adipate dihydrate form A is identical to the dihydrate prepared from wet organic solvent of medium polarity or by humidity exposure of anhydrous adipate.

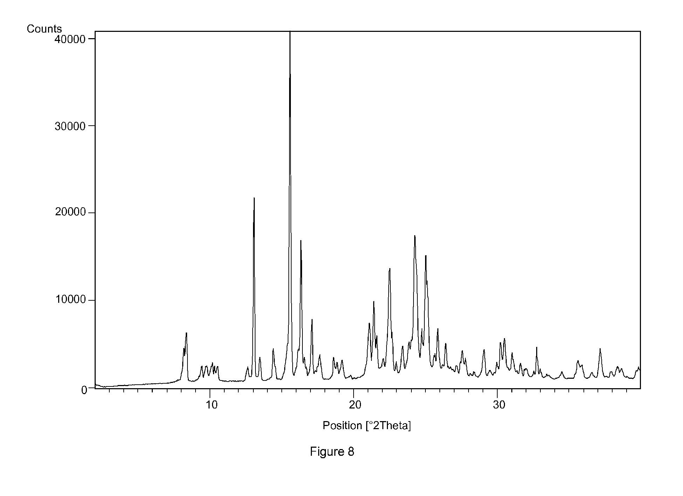

In another embodiment tamsulosin adipate, selected from dihydrate form A, anhydrous form or amorphous form is dissolved in a larger amount of water, preferably in an amount above 300 ml of water per gram of the salt, most preferably in 2000 ml of water per gram and the solution is let to environmental evaporation at room temperature for 1 to 5 weeks, preferably for 7 days. Resulted large crystals (monocrystals), which are isolated by decantation or filtration show X-Ray diffraction, characterised by the peaks at about 2θ= 13.1, 15.6, 17.1, 25.0, 27.6, 29.1 in 32.7° (Figure 8). The polymorph named tamsulosin adipate dihydrate form B has different crystal structure than the dihydrate prepared from wet organic solvent of medium polarity, from fast precipitation from water solutions, or by humidity exposure of anhydrous adipate. X-Ray analysis clearly shows two molecules of water in the crystal lattice and a hemiadipate structure with tamsulosin ―adipate ratio 2:1.

Tamsulosin adipate dihydrate is stable under normal ambient conditions but loses water in extremely dry conditions bellow 20 % of relative humidity preferably bellow 10 %. Dihydrate is transformed predominately into amorphous tamsulosin adipate, anhydrous crystalline tamsulosin adipate with identical XRPD data as the material prepared from anhydrous solvents is detected in a very minor extend or is not detected at all.

Tamsulosin adipate dihydrate loses water at higher temperatures, preferably above 70 °C, most preferably above 80 °C, again predominately amorphous material is formed and crystalline anhydrous tamsulosin adipate is detected only in traces or is not detected at all.

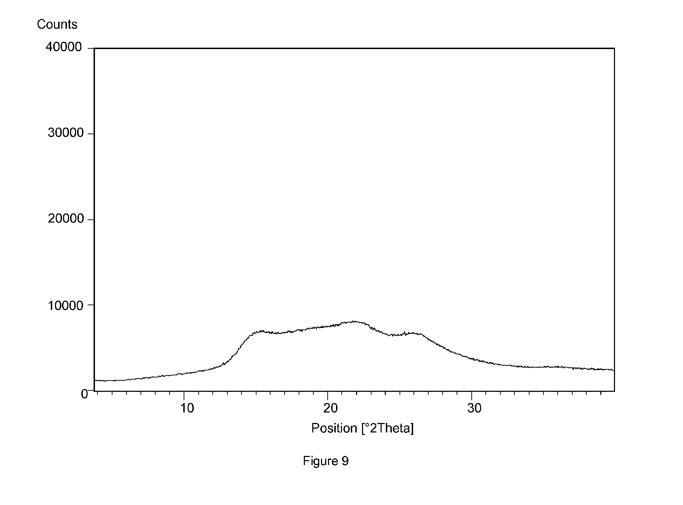

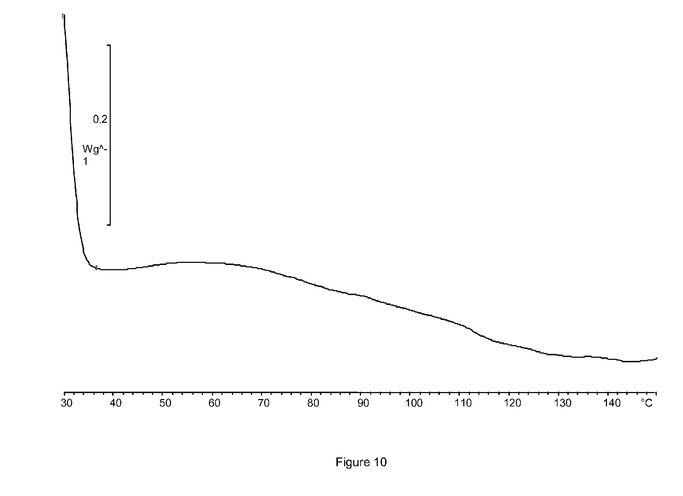

Amorphous tamsulosin adipate exhibits no peaks in XRD ― Figure 9 and no considerable changes in DSC ― Figure 10

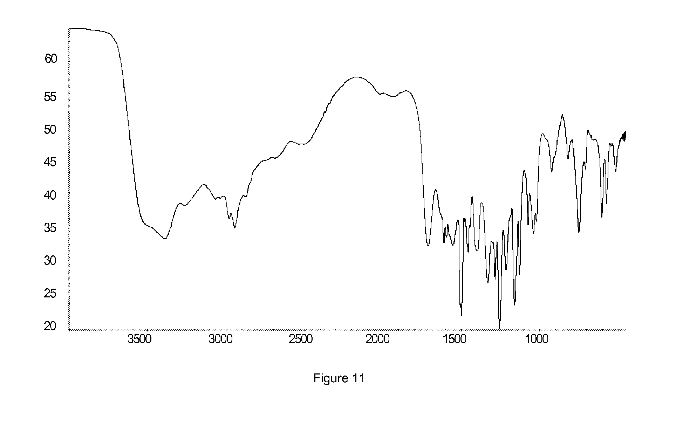

Amorphous tamsulosin adipate prepared by the process of the invention shows characteristic IR spectrum as depicted in Figure 11.

In a more applicable procedure the amorphous compound is prepared by liophylisation of water solutions of tamsulosin adipate.

The adipate forms of tamsulosin prepared according to the present invention have favourable solubility properties. Tamsulosin adipate is more soluble in lower alcohols, than the commercially available tamsulosin hydrochloride. This property could be an indicator for better interactions with less polar surfaces (e.g. lipid barriers) and allows more homogeneous preparation of pharmaceutical preparations in which tamsulosin should be homogenously mixed with lipophilic excipients (such as in preparation of transdermal dosage forms). The adipate anion has been shown physiologically acceptable. The tamsulosin adipate has therefore appropriate properties for smooth interaction with hydrophilic and lipophilic surfaces.

Tamsulosin adipate according to the invention can be used as an active ingredient in pharmaceutical finished dosage forms for treatment of benign prostatic hyperplasia, urinary incontinence and related conditions and disorders. The pharmaceutical formulation comprises a therapeutically effective amount of tamsulosin adipate together with a pharmaceutically acceptable diluent or carrier. As used herein, the term "effective amount" refers to the amount or dose of the compound which provides a desired effect in the patient under treatment, upon single or multiple dose administration. The pharmaceutical formulation can be administered orally in the form of a pellet, capsule, tablet, powder, granulate or any other suitable form, preferably in the form of pellets in capsules. The active ingredient and excipients may be formulated into pharmaceutical formulations according to methods known in the art. The pharmaceutical formulation of tamsulosin adipate can be administered also via other routes, such as parenteral (including subcutaneous, intramuscular and intravenous injections), topical (solutions, suspensions, emulsions, creams, ointments), inhalable, sublingual, buccal, rectal or transdermal formulations. Tamsulosin adipate can be used in formulations in which the release is extended or continuous, such as in the form of depo intramuscular injections. Preferably it is used transdermally.

Tamsulosin adipate dihydrate is favourable for routine preparation of final dosage forms with no special demands for anhydrous media because ambiental preparation can be applied. In the case of final dosage forms with reduced water content or with very lipophilic media the anhydrous crystalline adipate is preferred and the preparation in reduced humidity must be applied.

The products were analyzed by the following methods:
¹H-NMR spectroscopy:
   Tamsulosin / adipate ratio 1:1 was established by ¹H-NMR spectrum of a solution of sample in CDCl₃/ TMS, measured on Varian VNMRS 400 at 400 MHz with AutoX DB.
X-Ray powder diffraction (XRPD):
   The powder X-ray diffraction patterns were obtained by methods known in the art by using Philips X'Pert PRO diffractometer with X'Celerator detector using CuKa radiation (tube operating at 45 kV and 40 mA) in the Bragg-Brentano (reflection) geometry. The data were recorded from 2 to 40 °2θ in steps of 0.033 °2θ and the measurement time of 50 seconds per step. Variable divergence and antiscatter slits were used to maintain 12 mm of sample length irradiated.
Differential Scanning Calorimetry (DSC):
   DSC thermograms were obtained by using Mettler Toledo DSC822e differential scanning calorimeter. The sample (4-6 mg) was placed in an unsealed aluminium pan with a hole and heated at 5 °C/min in the temperature range from 30 °C to 200 °C.
IR spectroscopy:
   Fourier transform infrared (FTIR) spectra were recorded by using a Nicolet Nexus spectrometer. Spectra over a range of 4000 to 400 cm⁻¹ with a resolution of 2 cm⁻¹ (16 scans) were recorded on KBr tablets.

The present invention is illustrated but in no way limited by the following examples:

### Example 1

Tamsulosin base (1.0 g, 2.45 mmol) and acetonitrile (12 ml) are heated at reflux temperature until all solid is dissolved. Adipic acid (0.36 g, 2.46 mmol) is added to the solution and stirred until completely dissolved. Then the formed solution is slowly (in one hour) cooled down to 0 °C. An adhesive precipitate appears and diethyl ether (12 ml) is added. After standing at -14 °C for 12 h, the formed crystalline material is filtered, washed with fresh diethyl ether and dried in vacuum at 40 °C. 1.09 g of anhydrous tamsulosin adipate is obtained (80 % yield), m. p. 93-95 °C, DSC onset 103.77 °C.

### Example 2

Tamsulosin base (1.0 g, 2.45 mmol) and acetone (18 ml) are heated at reflux temperature until all solid is dissolved. Adipic acid (0.36 g, 2.46 mmol) is added to the solution and stirred until completely dissolved. Then the formed solution is slowly (in one hour) cooled down to 0 °C. An adhesive precipitate appears and diethyl ether (18 ml) is added. After standing at -16 °C for 15 h, the formed crystalline material is filtered, washed with fresh diethyl ether and dried in vacuum at 40 °C. 1.08 g of anhydrous tamsulosin adipate is obtained (79 % yield), m. p. 94-96 °C, DSC onset 103.89 °C.

### Example 3

Tamsulosin base (10.0 g, 24.48 mmol) and ethyl acetate (200 ml) are heated at reflux temperature until all solid is dissolved. Adipic acid (3.6 g, 24.63 mmol) is added to the solution and stirred until completely dissolved. Then the formed solution is slowly (in about two hours) cooled down to 0 °C. Spontaneously clear white solid is obtained and the mixture is diluted with diethyl ether (200 ml). The formed mixture is stirred for another two hours at 0 °C, filtered, washed with fresh diethyl ether and dried in vacuum at 40 °C. 23.46 g of anhydrous tamsulosin adipate is obtained (95.8 % yield), m. p. 95-97 °C, DSC onset 103.48 °C.

### Example 4

Tamsulosin base (10.0 g, 24.48 mmol) and acetonitrile (120 ml) are heated at reflux temperature until all solid is dissolved. Adipic acid (3.6 g, 24.63 mmol) is added to the solution and stirred until completely dissolved. After the formed solution is slowly (in two hours) cooled down to -10 °C, it becomes turbid. Methyl tert-butyl ether (120 ml) is added and the solution is stayed overnight at -15 °C. The formed crystalline material is precipitated, filtered, washed with fresh diethyl ether and dried in vacuum at 40 °C to give 10.92 g of anhydrous tamsulosin adipate (80 % yield), DSC onset 103.60 °C.

### Example 5

Tamsulosin base (50,0 g, 122 mmol) and ethyl acetate (1000 ml) are heated at reflux temperature until all solid is dissolved. Adipic acid (18,0 g, 123 mmol) is added to the solution and stirred until completely dissolved. Then the formed solution is slowly (in about two hours) cooled down to 0 °C. After a white solid is obtained, methyl tert-butyl ether (1000 ml) is added. The formed mixture is stirred for another two hours at 0 °C, filtered, washed with fresh methyl tert-butyl ether and dried in vacuum at 40 °C. 64.25 g (116 mmol) of anhydrous tamsulosin adipate is obtained (90.7 % yield), DSC onset 103.92 °C.

### Example 6

Tamsulosin base (5.02 g, 12.3 mmol) and ethyl acetate (100 ml) and adipic acid (1.8 g, 12.3 mmol) are heated at reflux temperature until all solid is dissolved then water (1.0 ml) is added and the formed solution is slowly (in about two hours) cooled down to 0 °C. After a white solid is obtained, methyl tert-butyl ether (100 ml) is added. If the precipitate sticks to the wall the mixture is optionally warmed and recooled. The suspension is stirred overnight at room temperature, the precipitate is then filtered, washed with fresh methyl tert-butyl ether and dried in vacuum at 40 °C to give 6.16 g (10.4 mmol) of tamsulosin adipate dihydrate form A with double peak on DSC (84,9 % yield, water content 5.97 %).

### Example 7

Tamsulosin base (5.02 g, 12.3 mmol) and ethyl acetate (100 ml) and adipic acid (1.8 g, 12.3 mmol) are heated at reflux temperature until all solid is dissolved followed by addition of first water (0.5 ml) and then diisopropyl ether (50 ml). The mixture is heated under reflux for further 30 min then slowly cooled. Bellow 60 °C first drops of oil are formed which soon solidify. The suspension is stirred overnight at room temperature, the precipitate is then filtered, washed with fresh diisopropyl ether and dried in vacuum at 40 °C to give 6.24 g (10.6 mmol) of tamsulosin adipate dihydrate form A with double peak on DSC (86.0 % yield, water content 5.78 %).

### Example 8

Tamsulosin base (5.02 g, 12.3 mmol) and ethyl acetate (100 ml) and adipic acid (1.8 g, 12.3 mmol) are heated at reflux temperature until all solid is dissolved followed by addition of first water (0.5 ml) and then diisopropyl ether (100 ml). Sticky precipitate appears and the mixture is rapidly cooled to 0 °C and the precipitate is homogenised by vigorous stirring. The stirring is continued for 2 days at room temperature, and the precipitate is then filtered, washed with fresh diisopropyl ether and dried in vacuum at 40 °C to give 6.06 g (10.3 mmol) of tamsulosin adipate for which XRD analysis shows a mixture anhydrous and dihydrate form of tamsulosin adipate (83.5 % yield, water content 5.59 %)

### Example 9

Anhydrous tamsulosin adipate from Example 3 (2.77 g, 5.0 mmol) is stayed in an open dish in a humidity chamber at temperature of 40 °C and relative humidity of 75 % for 14 days. The sample is homogenised and analysed to show a dihydrate form A with typical XRPD diffractogram, doubled peak on DSC and water content of 6.0 % .

### Example 10

Anhydrous tamsulosin adipate from Example 3 (300 mg) or amorphous tamsulosin adipate from Example 12 (300 mg) is dissolved in 25 ml of water at 25 °C to obtain a clear solution. Fine crystals are soon slowly precipitated during stirring. The solution is allowed at 25°C for 24 hours. The isolated crystals show diffraction pattern which corresponds to tamsulosin adipate dihydrate form A.

### Example 11

Tamsulosin adipate dihydrate from Example 6 (50 mg) is dissolved in 100 ml of water at 25 °C to obtain a clear solution. The solution is let to stand at 25 °C for 7 days. Precipitated monocrystals are separated from mother solution to give tamsulosin adipate dihydrate form B with characterised XRPD diffractogram shown in Figure 8.

### Example 12

Tamsulosin adipate dihydrate from Example 9 (1.0 g), is stayed in an open dish in an airtight chamber at temperature of 25 °C and relative humidity of below 10 % (achieved by means of dry desiccant) for 3 days. The sample which has got a foamy appearance is homogenised and analysed to show an amorphous material with plain curve on XRPD diffractogram. The weight loss (0.06 g) corresponds to the loss of water on transition from the crystalline dihydrate to the amorphous state.

### Example 13

Tamsulosin adipate dihydrate from Example 9 (1.0 g), is heated at 80 °C for 24 h to give a glassy solid which is crushed and homogenised. XRPD analysis shows an amorphous material with traces of anhydrous crystalline form.

### Example 14

Anhydrous tamsulosin adipate from Example 3 (300 mg) is dissolved in 25 ml of water. The solution is cooled by liquid nitrogen and the frozen mixture is lyophilised for 24 hours. Obtained product has characteristic diffractogram which shows completely amorphous material.

### Reference example A

Tamsulosin base (2.0 g, 4.9 mmol) is dissolved in methanol (16 ml) and adipic acid (0.72 g, 4.93 mmol) is added to the solution which is further slowly cooled down to 0 °C. No precipitate is formed. Diethyl ether (16 ml) is then added and the mixture is stayed overnight at -12 °C. An adhesive precipitate is formed which cannot be filtered. Solvents are evaporated to give yellowish oil which solidified after 5-10 days. The hard amorphous material is scratched off from the flask. There is no evidence that tamsulosin adipate would be formed.

### Reference example B

Tamsulosin base (1.0 g, 2.45 mmol) is dissolved in isopropanol (16 ml) and adipic acid (0.36 g, 2.46 mmol) is added to the solution. Then diethyl ether (16 ml) is added and the mixture is further stirred overnight at room temperature. A soft precipitate is filtered off, washed with fresh methyl tert-butyl ether and dried in vacuum at 40 °C. The solid material shows melting point of 129-131 °C, which is shown to be the melting point of tamsulosin base by adding a standard sample of the base.

## Claims

1. Tamsulosin adipate having the Formula 2: 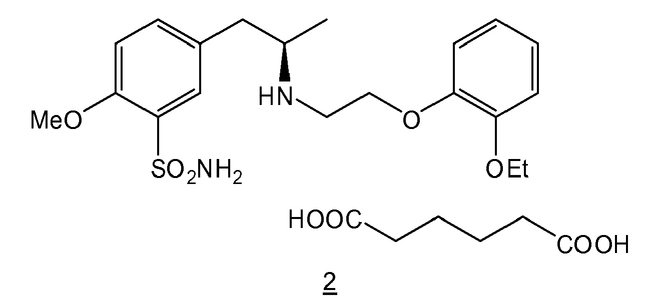 and the dihydrate thereof.

2. Tamsulosin adipate according to claim 1 **characterized by** one or more of XRD 2θ values at 10.0, 11.3, 13.8, 14.2, 17.2, 22.2, and 24.7°.

3. Tamsulosin adipate according to claim 1 **characterized by** the XRD diffractogram shown in the Figure 2.

4. Tamsulosin adipate according to claim 1 **characterized by** the melting point in the interval of 101-106 °C detected as an onset of endothermic transformation by DSC.

5. A process for the preparation of tamsulosin adipate by dissolving tamsulosin base and adipic acid in a solvent of a medium polarity.

6. The process according to claim 5 wherein said solvent is selected from ketones, nitriles and esters.

7. The process according to claim 6 wherein said solvent is acetone.

8. The process according to claim 6 wherein said solvent is acetonitrile.

9. The process according to claim 6 wherein said solvent is ethyl acetate.

10. The process according to any of claims 5 to 9 wherein said process is optionally followed by adding an antisolvent.

11. The process according to claim 10 wherein said antisolvent is an ether.

12. The process according to claim 11 wherein said ether is diethyl ether or methyl-tert-butyl ether.

13. A pharmaceutical formulation comprising a pharmaceutically effective amount of tamsulosin adipate as claimed in any of claims 1 to 4.

14. The pharmaceutical formulation according to claim 13 wherein said tamsulosin adipate is administered parenterally.

15. The pharmaceutical formulation according to claim 13 wherein said tamsulosin adipate is administered transdermally.
